# EUROPÄISCHE PATENTANMELDUNG

(11) **EP 4 763 455 A1**
(43) Veröffentlichungstag der Anmeldung: **24.06.2026**
(21) Anmeldenummer: 25222760.8
(22) Anmeldetag: 11.12.2025
(51) Int. Cl.: B27C 1/12

(54) **PENDELEINRICHTUNG FÜR MASCHINEN ZUR BEARBEITUNG VON WERKSTÜCKEN AUS HOLZ, KUNSTSTOFF UND DERGLEICHEN SOWIE MASCHINE MIT WENIGSTENS EINER PENDELEINRICHTUNG**

(30) Priorität: 18.12.2024 DE 102024004400
(71) Anmelder: Michael Weinig AG, 97941 Tauberbischofsheim (DE)
(72) Erfinder: BLESCH, Lucas, 74744 Ahorn-Eubigheim (DE); FRANK, Benjamin, 97944 Boxberg (DE); KUHN, Andreas, 97318 Kitzingen (DE); WAGNER, Ralf, 74736 Hardheim (DE)
(74) Vertreter: Patentanwälte Jackisch-Kohl und Kohl

(57) **Zusammenfassung**

Die Pendeleinrichtung für Maschinen zur Bearbeitung von Werkstücken aus Holz, Kunststoff und dergleichen hat wenigstens eine Vorschubwalze (10), die an einem Pendel drehbar gelagert ist. An ihm ist wenigstens ein Zylinder (29) vorgesehen, in dem ein Kolben (30) verschiebbar ist. Um die Pendeleinrichtung und eine Maschine mit wenigstens einer solchen Pendeleinrichtung so auszubilden, dass die Schwingbewegung der Vorschubwalzen (10) in konstruktiv einfacher Weise innerhalb kürzester Zeit beendet ist, trennt der Kolben (30) einen Luftraum (32) von einem Ölraum (31), der mit einem Ölraum (42) eines Druckmittelwandlers (38) leitungsverbunden ist.

## Beschreibung

Die Erfindung betrifft eine Pendeleinrichtung für Maschinen zur Bearbeitung von Werkstücken aus Holz, Kunststoff und dergleichen nach dem Oberbegriff des Anspruches 1 sowie eine Maschine mit wenigstens einer solchen Pendeleinrichtung nach Anspruch 9.

Bei Bearbeitungsmaschinen, insbesondere Holzbearbeitungsmaschinen, insbesondere Kehlmaschinen, werden die Werkstücke mit Hilfe von Vorschubwalzen durch die Maschine geführt. Die Vorschubwalzen sind in Pendeln drehbar gelagert, an denen jeweils eine Stelleinheit vorgesehen ist. Die Stelleinheit weist einen Pneumatikzylinder und eine Einrichtung auf, mittels derer die Vorschubwalzen in einer Grundstellung so eingestellt sind, dass die zu bearbeitenden Werkstücke die Vorschubwalzen bei ihrem Transport durch die Maschine anheben. Dabei ist die Vorschubwalze über den mit einem Druckmedium beaufschlagten Pneumatikzylinder in Richtung des Werkstücks belastet. Da die Werkstücke mit hoher Geschwindigkeit transportiert werden, werden die Vorschubwalzen schlagartig nach oben ausgelenkt und hochgeschleudert. Durch das Hochschleudern heben sie zunächst von dem Werkstück ab, um dann wieder auf dieses zurückzufallen. Dieses erfolgt quasi als elastischer Stoß, wodurch die Vorschubwalze wieder nach oben geschleudert wird und erneut von dem Werkstück abhebt. Dieser Vorgang wiederholt sich einige Male, bis die Vorschubwalzen ruhig auf dem Werkstück aufliegen. Das bedeutet, dass diese Schwing- bzw. Pendelbewegung der Vorschubwalzen mit der Zeit abklingt, so dass die Vorschubwalzen auf den Werkstücken aufliegen und diese transportieren können. Die Schwingbewegung der Vorschubwalzen wird erst nach einem längeren Transportweg der Werkstücke beendet.

Durch dieses mehrmalige Abheben vom Werkstück und wieder Auftreffen wird zum einen der Transport der Werkstücke negativ beeinflusst. Zum anderen sind die Werkstücke im Moment des Abhebens nicht mehr sauber gehalten und werden beim Auftreffen der Vorschubwalze auf das Werkstück zu Schwingungen angeregt, was sich in beiden Fällen negativ auf die Oberflächenqualität bei der Bearbeitung auswirkt.

Der Erfindung liegt die Aufgabe zugrunde, die gattungsgemäße Pendeleinrichtung und die Maschine so auszubilden, dass die Schwingbewegung der Vorschubwalzen in konstruktiv einfacher Weise innerhalb kürzester Zeit beendet ist.

Diese Aufgabe wird bei der gattungsgemäßen Pendeleinrichtung erfindungsgemäß mit den kennzeichnenden Merkmalen des Anspruches 1 und bei der Maschine mit den Merkmalen des Anspruches 9 gelöst.

Bei der erfindungsgemäßen Pendeleinrichtung ist der Kolben des Zylinders so vorgesehen, dass er zwischen dem Luftraum und dem Ölraum liegt. Wird die Vorschubwalze durch das Werkstück angehoben, bewegt sich der Kolben in Richtung auf den Ölraum, der mit dem Ölraum des Druckmittelwandlers leitungsverbunden ist. Durch die Aufwärtsbewegung des Kolbens wird das Hydraulikmedium aus dem Ölraum des Zylinders in den Ölraum des Druckmittelwandlers verdrängt. Dabei gelangt das verdrängte Hydraulikmedium nicht schlagartig in den Druckmittelwandler, sondern infolge einer Drosselwirkung der Leitungsverbindung zwischen den beiden Ölräumen nur mit begrenzter Strömungsgeschwindigkeit. Dadurch wird eine sehr hohe Dämpfungswirkung erzielt, die dazu führt, dass die Vorschubwalze beim Kontakt mit dem Werkstück nicht mehr so stark nach oben geschleudert wird und die Schwingungs-/Pendelbewegung innerhalb kürzester Zeit abklingt. Dadurch wird der Transport der Werkstücke durch die Maschine wesentlich verbessert. Die Schwingung des Pendels überträgt sich nicht auf das Werkstück, so dass eine hohe Laufruhe erzeugt wird. Sie wiederum ist entscheidend für die Qualität der Bearbeitung des Werkstückes.

Als Hydraulikmedium kommen nicht nur Öle bzw. Hydrauliköle in Betracht, sondern auch andere Hydraulikfluide, wie Hydraulikflüssigkeiten auf Wasserbasis. Wenn von Ölräumen die Rede ist, ist dies daher nicht als Beschränkung auf Öle zu verstehen.

Vorzugsweise steht das im Ölraum des Druckmittelwandlers befindliche Hydraulikmedium unter Druck. Dadurch wirkt eine Kraft auf das Vorschubpendel, welche sich durch Änderung des Drucks einstellen lässt.

Als Druckmedium wird in vorteilhafter Weise Gas, insbesondere Druckluft, eingesetzt. Druckluft steht üblicherweise zur Verfügung. Sie ist ein einfach zu handhabendes Medium.

Zur Erzielung des gewünschten Dämpfungsverhaltens trägt in vorteilhafter Weise bei, dass in der Leitungsverbindung zwischen dem Ölraum des Zylinders und dem Ölraum des Druckmittelwandlers eine Drosselstelle für das Hydraulikmedium vorgesehen ist. Die Drosselstelle sorgt dafür, dass das Hydraulikmedium nur in begrenztem Maße verdrängt werden kann, was sich vorteilhaft auf die Dämpfungswirkung auswirkt.

Die Pendeleinrichtung weist vorteilhaft zwei Vorschubwalzen auf, deren Pendel gelenkig angeordnet und jeweils mit einer Stelleinheit verbunden sind.

In diesem Falle ist es besonders vorteilhaft, wenn beide Zylinder der beiden Stelleinheiten an einen gemeinsamen Druckmittelwandler angeschlossen sind. Dies ergibt einen konstruktiv sehr einfachen Aufbau der Pendeleinrichtung.

Es ist beispielhaft möglich, dass auf ein Pendel zwei Zylinder wirken und damit bei zwei Pendeln vier Zylinder an den Druckmittelwandler angeschlossen sind.

Es ist von Vorteil, wenn die Leitungsverbindung zwischen den Ölräumen der Stelleinheit und des Druckmittelwandlers einen Schlauch aufweist. Er ist flexibel und ermöglicht die Leitungsverbindung zwischen den beiden Einheiten unabhängig von ihrem Einbauort in der Pendeleinrichtung.

Bei einer besonders vorteilhaften Ausbildung wird die Drosselstelle durch den Anschluss des Schlauches an den Zylinder gebildet.

Die erfindungsgemäße Maschine zeichnet sich dadurch aus, dass sie mit wenigstens einer erfindungsgemäßen Pendeleinrichtung ausgestattet ist. Die Maschine ermöglicht dadurch eine optimale Transportführung der Werkstücke, so dass keine Qualitätseinschränkungen an der Werkstückoberseite auftreten.

Eine besonders vorteilhafte Ausbildung der Maschine ist eine Kehlmaschine, mit der die Werkstücke bei ihrem Durchlauf an allen vier Seiten bearbeitet werden können.

Vorteilhaft wird das gedämpfte Vorschubpendel im Einlauf der Kehlmaschine eingesetzt. Hier können durch die Rohholzunterschiede die Pendel in Abhängigkeit von der Transportgeschwindigkeit besonders stark ausgelenkt werden. Letztendlich ist der Zusammenhang Rohholzübermaß und Transportgeschwindigkeit entscheidend. Es kann aber auch innerhalb der Maschine, wo die Werkstücke schon teilweise bearbeitet sind von Vorteil sein, gedämpfte Vorschubpendel einzusetzen, z.B. vor jeder Bearbeitungsspindel.

Der Anmeldungsgegenstand ergibt sich nicht nur aus dem Gegenstand der einzelnen Patentansprüche, sondern auch durch alle in den Zeichnungen und der Beschreibung offenbarten Angaben und Merkmale. Sie werden, auch wenn sie nicht Gegenstand der Ansprüche sind, als erfindungs-wesentlich beansprucht, soweit sie einzeln oder in Kombination gegenüber dem Stand der Technik neu sind.

Weitere Merkmale der Erfindung ergeben sich aus den weiteren Ansprüchen, der Beschreibung und den Zeichnungen.

Die Erfindung wird anhand eines in den Zeichnungen dargestellten Ausführungsbeispieles näher erläutert. Es zeigen
- Fig. 1: in perspektivischer Darstellung einen Teil einer erfindungsgemäßen Bearbeitungsmaschine mit einer erfindungsgemäßen Pendeleinrichtung,
- Fig. 2: in Vorderansicht die erfindungsgemäße Pendeleinrichtung gemäß Fig. 1,
- Fig. 3: die erfindungsgemäße Pendeleinrichtung gemäß Fig. 2 in perspektivischer Darstellung,
- Fig. 4: ein Pendelweg-Werkstückweg-Diagramm.

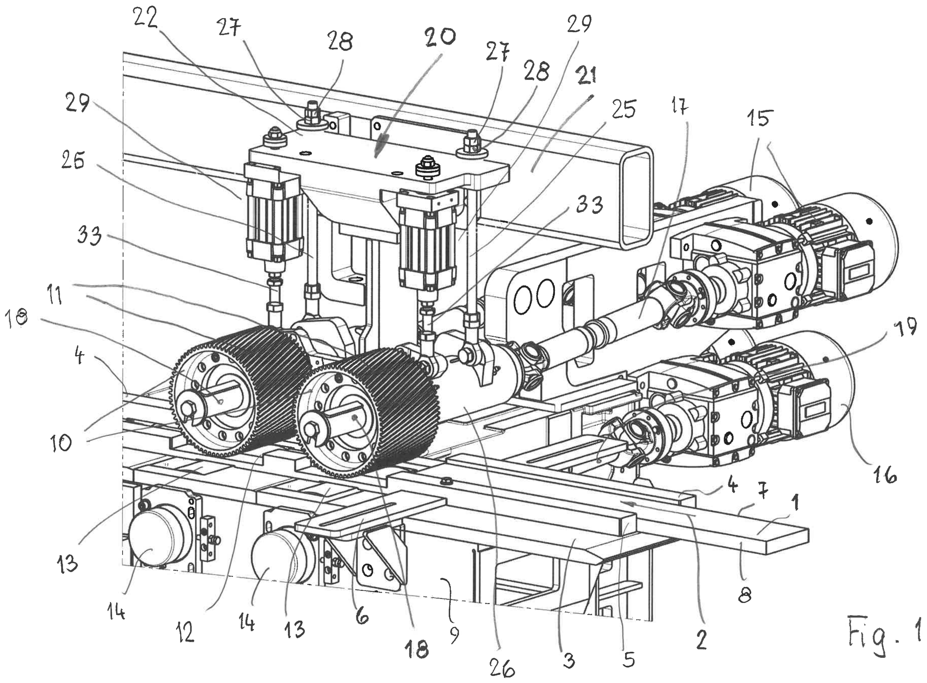

Die Bearbeitungsmaschine dient zur Bearbeitung von Werkstücken aus Holz oder holzähnlichen Materialien. Die Werkstücke 1 sind in Form von Brettern ausgebildet und haben beispielhaft rechteckigen Querschnitt (Fig. 1). Die Werkstücke 1 werden in ihrer Längsrichtung in Richtung des Pfeiles 2 durch die Bearbeitungsmaschine transportiert. Bei ihrem Durchlauf durch die Maschine werden die Werkstücke 1 in geeigneter Weise bearbeitet.

Im Ausführungsbeispiel ist die Bearbeitungsmaschine eine Kehlmaschine, mit der die Werkstücke 1 bei ihrem Durchlauf durch die Maschine an allen vier Seiten bearbeitet werden.

Die zu bearbeitenden Werkstücke 1 werden auf einem Einlauftisch 3 zugeführt, der aus einzelnen Tischplatten bestehen kann.

Auf dem Einlauftisch 3 befinden sich ein in Transportrichtung 2 rechtes Anschlaglineal 4 und ein linkes Anschlaglineal 5. Die beiden Anschlaglineale 4, 5 erstrecken sich parallel zueinander in Transportrichtung 2.

Das linke Anschlaglineal 5 kann zur Anpassung an unterschiedlich breite Werkstücke 1 senkrecht zur Transportrichtung 2 verstellt werden. Die entsprechende Einstelleinrichtung 6 ist in Fig. 1 schematisch dargestellt.

Die Werkstücke liegen bei ihrem Transport durch die Maschine mit ihren parallelen Längsseiten 7, 8 an den Anschlaglinealen 4, 5 an, welche das Werkstück 1 bei seinem Durchlauf durch die Maschine einwandfrei führen. Dadurch kann das Werkstück 1 sauber durch entsprechende Werkzeuge bearbeitet werden.

Der Einlauftisch 3 mit den Anschlaglinealen 4, 5 und der Einstelleinrichtung 6 befindet sich auf einem Ständer 9, der an einem nicht dargestellten Maschinenständer zur Einstellung einer Spanabnahme an einem ebenfalls nicht dargestellten ersten unteren Werkzeug höhenverstellbar gelagert ist.

Zum Transport der Werkstücke 1 durch die Maschine dienen Vorschubwalzen 10, die in Transportrichtung 2 in der Maschine mit Abstand hintereinander liegen und die unter Kraft auf den Werkstücken 1 aufliegen. Die Vorschubwalzen 10 werden um ihre Achse drehbar angetrieben und haben vorteilhaft an ihrer Mantelfläche eine Riffelung oder Verzahnung 11, die einen sicheren Transport der Werkstücke 1 durch die Vorschubwalzen 10 gewährleistet.

In Höhe der Vorschubwalzen 10 befinden sich im Bereich unterhalb einer Transportbahn 12 für die Werkstücke 1 um horizontale Achsen drehbare Tischwalzen 13, die an der Unterseite der Werkstücke 1 bei ihrem Durchlauf durch die Maschine anliegen und zum Vorschub der Werkstücke 1 durch die Maschine beitragen.

Die Achsen der Vorschubwalzen 10 und der Tischwalzen 13 verlaufen horizontal und senkrecht zur Transportrichtung 2.

Zum Antrieb der Vorschubwalzen 10 und der Tischwalzen 13 sind Antriebsmotoren 15 und 16 vorgesehen, die in geeigneter Weise am Maschinenständer befestigt sind. Die Antriebsmotoren 15 sind über Kardangelenkwellen 17 mit Wellen 18 antriebsverbunden, auf denen die Vorschubwalzen 10 drehfest sitzen.

Auch die Antriebsmotoren 16 sind über Kardangelenkwellen 19 mit (nicht dargestellten) Wellen antriebsverbunden, auf denen die Tischwalzen 13 drehfest sitzen.

Die Kardangelenkwellen 17, 19 ermöglichen die Höhenverstellung der Vorschubwalzen 10 und der Tischwalzen 13 zur Anpassung an unterschiedlich dicke Werkstücke 1. Die Tischwalzen 13 sind im Ständer 9 des Einlauftisches 3 gelagert und zur Maschinenvorderseite hin jeweils mit einer Abdeckung 14 versehen.

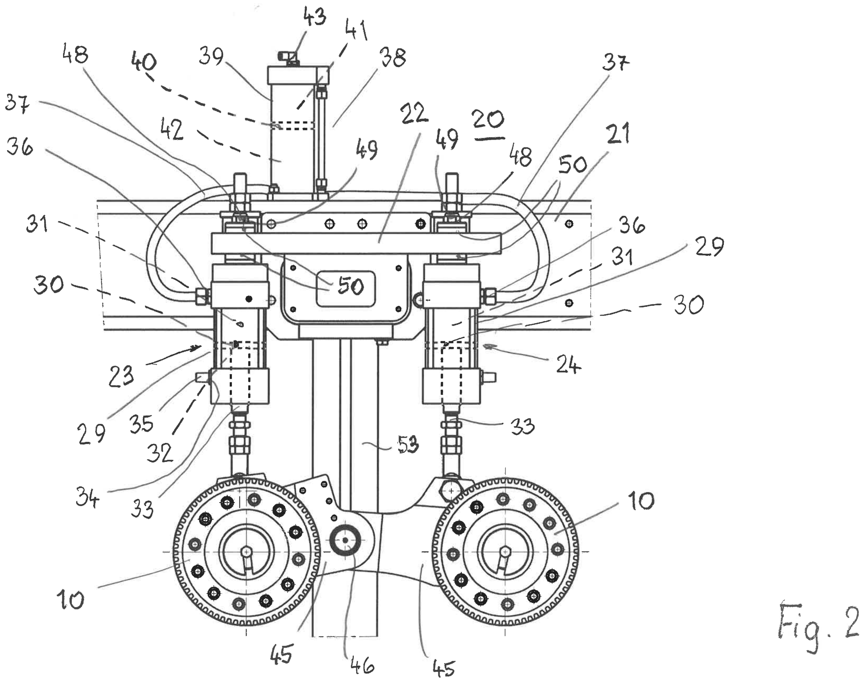

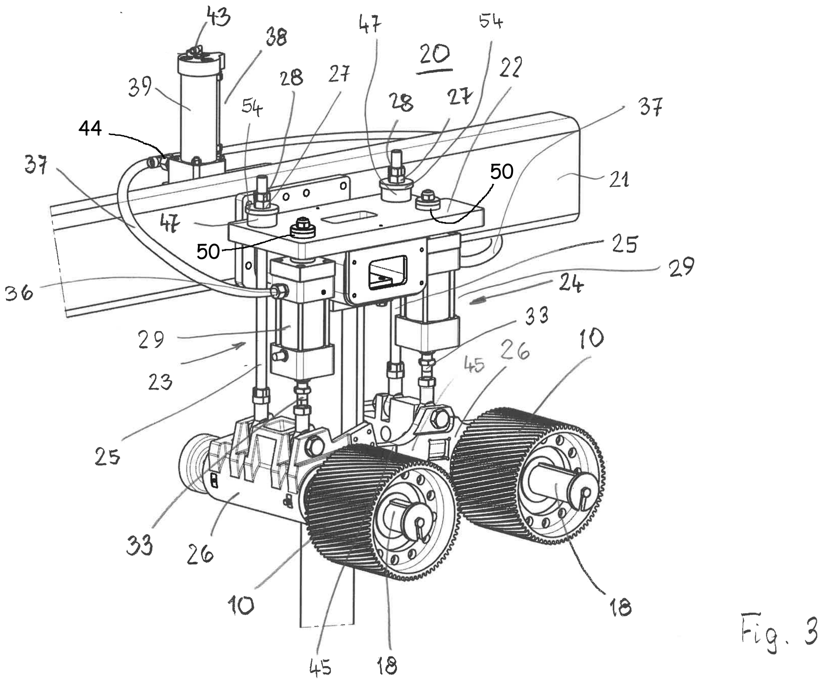

Die Vorschubwalzen 10 sind Bestandteil einer Pendeleinrichtung 20, die in den Fig. 2 und 3 dargestellt ist. In Fig. 1 ist der Übersichtlichkeit wegen nur ein Teil der Pendeleinrichtung 20 dargestellt. Die Pendeleinrichtung 20 ist an einem Transportbalken 21 aufgehängt, der sich mit Abstand oberhalb der Transportbahn 12 befindet und sich in Transportrichtung 2 der Werkstücke 1 erstreckt. Am Transportbalken 21 ist eine Konsole 22 befestigt, die quer vom Transportbalken 21 absteht und an der zwei Stelleinheiten 23, 24 gelagert sind, mit denen die Vorschubwalzen 10 in Höhenrichtung verstellt und mit einer Kraft belastet werden können. Da beide Stelleinheiten 23, 24 gleich ausgebildet sind, wird nachfolgend die Stelleinheit 23 näher erläutert.

Sie hat eine Stellstange 25, die mit ihrem oberen Ende durch eine Bohrung der plattenförmigen Konsole 22 ragt und deren unteres Ende mit einem Pendel 26 gelenkig verbunden ist. Das Pendel 26 bildet eine Lagerung für die Welle 18 der Vorschubwalze 10.

Die Stellstange 25 ist in bekannter Weise verschiebbar in der Konsole 22 gelagert, so dass durch entsprechende axiale Verstellung der Stellstange 25 gegenüber der Konsole 22 der Abstand des Pendels 26 und damit der Vorschubwalze 10 zur Transportbahn 12 eingestellt werden kann. Zur Axialsicherung der Stellstange 25 sind eine Mutter 27 und eine Kontermutter 28 vorgesehen, die auf dem über die Konsole 22 überstehenden Ende der Stellstange 25 sitzen.

Mit der Stellstange 25 wird die Grundstellung der Pendel 26 und damit der Vorschubwalzen 10 eingestellt.

Neben der Stellstange 25 befindet sich ein Zylinder 29, der an der den Vorschubwalzen 10 zugewandten Unterseite der Konsole 22 gehalten ist und von ihr nach unten senkrecht absteht. Im Zylinder 29 ist ein Kolben 30 untergebracht, der einen Ölraum 31 von einem Luftraum 32 trennt.

Der Kolben 30 ist mit einer Kolbenstange 33 verbunden, die den Luftraum 32 durchsetzt und abgedichtet durch den Boden des Zylinders 29 ragt. Die Kolbenstange 33 ist gelenkig mit dem Pendel 26 verbunden.

In den Luftraum 32 mündet eine Öffnung 34, über die Luft aus dem Luftraum 32 nach außen bzw. Außenluft nach innen in den Luftraum 32 strömen kann. An die Öffnung 34 ist vorteilhaft ein Schalldämpfer 35 angeschlossen, der vom Zylinder 29 absteht und dafür sorgt, dass beim Durchtritt der Luft durch die Öffnung 34 keine störenden Schallgeräusche auftreten.

Im Ölraum 31 des Zylinders 29 befindet sich vorteilhaft Hydrauliköl, das als Dämpfungsmedium für die Pendelbewegung der Vorschubwalzen 10 beim Auftreffen auf die Werkstücke 1 in noch zu beschreibender Weise dient.

In den Ölraum 31 mündet ein Anschluss 36, der über einen Ölschlauch 37 mit einem Druckmittelwandler 38 leitungsverbunden ist. Er ist am Transportbalken 21 befestigt und hat ein Gehäuse 39, in dem eine Beruhigungsscheibe 40 untergebracht ist. Sie trennt einen Druckluftraum 41 von einem Ölraum 42. Er ist über den Ölschlauch 37 mit dem Ölraum 31 des Zylinders 29 verbunden.

Das Gehäuse 39 hat einen Druckluftanschluss 43, an den ein Druckluftschlauch anschließbar ist. Er verbindet den Druckluftraum 41 mit einer (nicht dargestellten) Druckluftquelle.

Der Anschluss 36 wirkt als Drosselstelle, welche das Maß der Dämpfung der Schwingungsbewegung der Vorschubwalzen 10 bestimmt.

Der Druckmittelwandler 38 ist in der beschriebenen Weise über die Ölschläuche 37 mit beiden Zylindern 29 der Stelleinheiten 23, 24 der Pendeleinrichtung 20 verbunden.

Die Maschine weist über ihre Länge mehrere Pendeleinrichtungen 20 auf, die in der beschriebenen Weise ausgebildet sind. Jede Pendeleinrichtung 20 weist zwei parallel zueinander liegende Vorschubwalzen 10 auf, die auf den Wellen 18 der Pendel 26 sitzen. Die beiden Pendel 26 haben zueinander gerichtete Ausleger 45. Die Pendel 26 haben an den freien Enden der Ausleger 45 durch eine Achse 46 gelenkig in einem Transportschieber 53 gelagert. Die Achse 46 verläuft parallel zu den Wellen 18 im Bereich zwischen den beiden Vorschubwalzen 10 (Fig. 2).

An den beiden Pendeln 26 greifen jeweils die unteren Enden der Stellstangen 25 und der Kolbenstangen 33 gelenkig an. Zwischen den Muttern 27 der Stellstangen 25 und der Oberseite der Konsole 22 sind Scheiben 54 und Dämpfungsscheiben 47 angeordnet. Die Dämpfungsscheiben 47 umgeben das über die Konsole 22 nach oben überstehende Ende der Stellstangen 25, die gegenüber der Dämpfungsscheibe 47 verschiebbar sind. In der Ausgangslage liegen die Muttern 27 der Stellstangen 25 unter dem Gewicht der Pendel 26 mit den Vorschubwalzen 10 unter Zwischenlage der Scheibe 54 auf den Dämpfungsscheiben 47 auf. Zusätzlich wirkt die über den Pneumatikdruck eingestellte Zylinderkraft in gleicher Richtung.

Die Zylinder 29 haben an ihrer Oberseite einen axial vorstehenden Bolzen 48, der die Konsolen 22 durchsetzt und auf dessen freiem Ende eine Mutter 49 sitzt. Der Zylinder 29 ist unter Zwischenlage von Dämpfungsscheiben 50 an der Konsole 22 angebracht. Die Dämpfungsscheiben 50 umgeben den Bolzen 48.

Die Stellstangen 25 und die Zylinder 29 mit den Kolbenstangen 33 liegen jeweils mit Abstand hintereinander und sind in der beschriebenen Weise jeweils gelenkig mit den Pendeln 26 verbunden.

Die Vorschubwalzen 10 sind mit Hilfe der Stellstangen 25 auf eine Grundstellung eingestellt, die abhängig ist von der Dickentoleranz der durch die Maschine zu transportierenden rohen Werkstücke 1. Die Grundstellung ist so gewählt, dass die Werkstücke 1 beim Auftreffen auf die Transportwalzen 10 diese aus der Grundstellung anheben. Dies hat zur Folge, dass die Stellstangen 25 über die Pendel 26 hierbei axial gegenüber der Konsole 22 verschoben werden, so dass die Muttern 27 auf den Stellstangen 25 von den Dämpfungsscheiben 47 abheben. Gleichzeitig werden auch die Kolbenstangen 33 relativ zu den Zylindern 29 verschoben. Die beiden Vorschubwalzen 10 sind wie beschrieben über die Achse 46 gelenkig angeordnet und werden gegeneinander um die Achse 46 verschwenkt, wenn das Werkstück 1 beim Transport durch die Maschinen nacheinander die Vorschubwalzen 10 erreicht. Dadurch wird erreicht, dass die Vorschubwalzen stets auf dem Werkstück 1 aufliegen und es zuverlässig durch die Maschine transportieren.

Sobald die Werkstücke 1 mit ihrem in Transportrichtung 2 vorderen Ende auf die erste Vorschubwalze 10 treffen, wird diese nach oben ausgelenkt.

Je nach Rohholzübermaß und, da die Werkstücke 1 mit verhältnismäßig hoher Geschwindigkeit durch die Maschinen transportiert werden, werden die Vorschubwalzen 10 nicht kontinuierlich aufwärtsbewegt, sondern hochgeschleudert. Dies hat zur Folge, dass die Vorschubwalzen 10 eine Art Pendelbewegung ausführen.

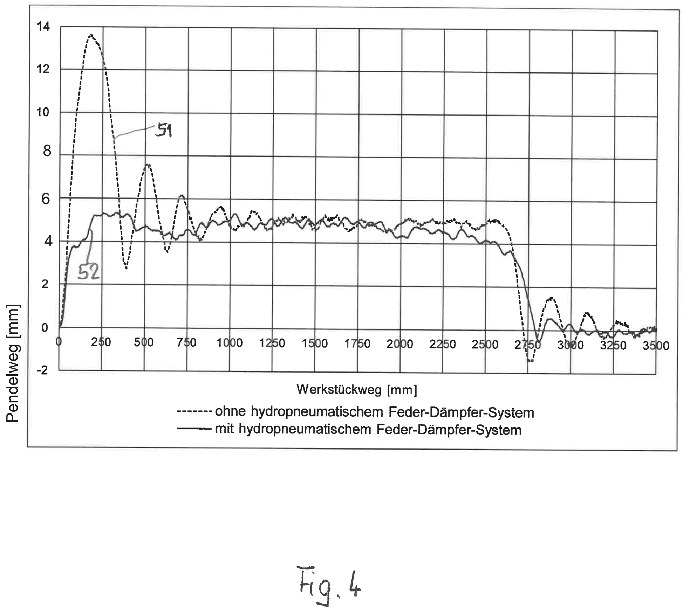

In Fig. 4 ist das Pendelverhalten der Vorschubwalzen 10 dargestellt. Der Pendelausschlag ist gegen den Werkstückweg dargestellt. Die Kurve 51 zeigt das Pendelverhalten der Vorschubwalzen bei den herkömmlichen Maschinen ohne Dämpfung. Erkennbar ist ein sehr hoher Pendelausschlag, wenn das Werkstück 1 auf die Vorschubwalzen 10 trifft. Es sind weitere abnehmende Pendelausschläge erkennbar, bis die Vorschubwalze 10 auf dem Werkstück 1 ruhig aufliegt.

Sobald das Werkstück 1 die Vorschubwalze 10 passiert hat, fällt die Vorschubwalze schlagartig nach unten in Richtung auf ihre Grundstellung. Auch hier treten erhebliche Pendelausschläge auf, bis die Vorschubwalze ihre durch die Stellstange 25 definierte Grundstellung einnehmen kann.

Demgegenüber zeigt die Kurve 52 das Pendelverhalten der erfindungsgemäßen Ausbildung. Die Pendelausschläge sind wesentlich geringer sowohl beim Auftreffen des Werkstückes 1 auf die Vorschubwalze als auch beim Herabfallen nach dem Passieren des Werkstückes 1. Erreicht wird diese sehr hohe Dämpfung durch die beschriebene Gestaltung der Zylinder 29 in Verbindung mit dem Druckmittelwandler 38.

Sobald das Werkstück 1 auf die in Fig. 2 rechte Vorschubwalze 10 trifft, wird diese aus ihrer durch die Stellstange 25 festgelegten Grundstellung nach oben ausgehoben. Dies hat zur Folge, dass über die Kolbenstange 33 der Kolben 30 innerhalb des Zylinders 29 aufwärts verschoben wird. Hierbei wird das im Ölraum 31 befindliche Öl über den Anschluss 36 in den Ölschlauch 37 und von dort in den Ölraum 42 des Druckmittelwandlers 38 verdrängt. Der Ölschlauchanschluss 36 bildet beim Verdrängen des Öls aus dem Ölraum 31 eine Drosselstelle, so dass das Öl im Ölraum 31 nur mit für den Anwendungsfall begrenzter idealer Strömungsgeschwindigkeit in den Ölraum 42 des Druckmittelwandlers 38 verdrängt werden kann. Der Anschluss 36 stellt die Hauptdrosselstelle dar. Ergänzend haben auch der Anschluss 44 des Druckmittelwandlers 38 und der Ölschlauch 37 eine Drosselwirkung.

In Verbindung mit der Ölviskosität des verwendeten Hydrauliköls entsteht über diese Drosselstellen im Hydraulikkreislauf das gute Dämpfungsverhalten (siehe Kurve 52 in Fig. 4). Die Beruhigungsscheibe 40 liegt auf dem Öl auf und verhindert die Schaumbildung im Öl und ein Aufschwappen des in den Ölraum 42 verdrängten Öls.

Die im Druckluftraum 41 befindliche Druckluft hält das im Ölraum 42 befindliche Öl stets unter dem eingestellten Druck. Über die Einstellung des Pneumatikdrucks kann die Auflagekraft der Vorschubwalzen in Abhängigkeit der zu bearbeitenden Werkstücke 1 optimal vorgegeben werden. Außerdem sorgt die Druckluft für eine schnelle Rückstellung der Pendel in die Grundstellung.

Um das Öl im Ölraum 42 des Druckmittelwandlers 38 unter stetem Druck zu halten, kann anstelle der Druckluft beispielhaft auch wenigstens eine Druckfeder eingesetzt werden, die sich im Druckraum 41 befindet und in geeigneter Weise das Öl im Ölraum 42 unter Druck setzt.

Beim Anheben der Vorschubwalze 10 wird der Kolben 30 im Zylinder 29 aufwärtsbewegt. Über den Anschluss 34 wird Außenluft nachgezogen.

Umgekehrt wird die im Luftraum 32 befindliche Luft beim Absenken des Kolbens 30 durch den Anschluss 34 nach außen verdrängt. Im gleichen Maße wird über den Anschluss 36 und den Ölschlauch 37 Öl aus dem Ölraum 42 des Druckmittelwandlers 38 nachgeführt. Das hydropneumatische System sorgt dafür, dass die Pendelbewegungen der Vorschubwalze 10 in der beschriebenen Weise sehr gedämpft erfolgt. Die gute Dämpfungseigenschaften resultieren daraus, dass ein Hydraulikmedium aus einem zylinderseitigen Ölraum 31 in einen Ölraum 42 eines Druckmittelwandlers 38 über Drosselstellen im Hydraulikkreislauf geleitet wird. Dabei wird die Bewegungsenergie des Hydraulikmediums in Wärmeenergie umgewandelt. Der Dämpfungsgrad ergibt sich über die als Drosselstellen wirkenden Zylinderanschlüsse bzw. Verschraubungen 36, 44 und die gewählten Durchmesser und Längen der Hydraulikschläuche 37 in Verbindung mit der Ölviskosität des Hydraulikmediums. Über diese Parameter kann eine gewünschte Dämpfung vorgegeben werden. Wie die Kurve 52 in Fig. 4 zeigt, sind die Ausschlagbewegungen der Vorschubwalze 10 sehr gering, wenn das Werkstück 1 unter der Transportwalze 10 hindurch transportiert worden ist.

Der Zylinder 29 ist ein Hydraulikzylinder, vorzugsweise ein Niederdruckhydraulikzylinder. Er hat eine spezielle Dichtung, die den Ölraum von dem Luftraum zuverlässig trennt und abdichtet.

## Patentansprüche

1. Pendeleinrichtung für Maschinen zur Bearbeitung von Werkstücken aus Holz, Kunststoff und dergleichen, mit wenigstens einer Vorschubwalze (10), die an einem Pendel (26) drehbar gelagert ist, an dem wenigstens ein Zylinder (29) vorgesehen ist, in dem ein Kolben (30) verschiebbar ist,
**dadurch gekennzeichnet, dass** der Kolben (30) einen Luftraum (32) von einem Ölraum (31) trennt, der mit einem ein Hydraulikmedium enthaltenden Ölraum (42) eines Druckmittelwandlers (38) leitungsverbunden ist.

2. Pendeleinrichtung nach Anspruch 1,
**dadurch gekennzeichnet, dass** das im Ölraum (42) des Druckmittelwandlers (38) befindliche Hydraulikmedium unter Druck steht.

3. Pendeleinrichtung nach Anspruch 1 oder 2,
**dadurch gekennzeichnet, dass** in einem Druckraum (41) des Druckmittelwandlers (38) Gas, vorzugsweise Druckluft, als Druckmedium eingesetzt wird.

4. Pendeleinrichtung nach einem der Ansprüche 1 bis 3,
**dadurch gekennzeichnet, dass** in der Leitungsverbindung zwischen dem Ölraum (31) des Zylinders (29) und dem Ölraum (43) des Druckmittelwandlers (38) eine Drosselstelle (36, 37, 44) für das Hydraulikmedium vorgesehen ist.

5. Pendeleinrichtung nach einem der Ansprüche 1 bis 4,
**dadurch gekennzeichnet, dass** die Pendeleinrichtung zwei Vorschubwalzen (10) aufweist, deren Pendel (26) gelenkig angeordnet und mit jeweils einer Stelleinheit (23, 24) verbunden sind.

6. Pendeleinrichtung nach Anspruch 5,
**dadurch gekennzeichnet, dass** beide Zylinder (29) der beiden Stelleinheiten (23, 24) an einen gemeinsamen Druckmittelwandler (38) angeschlossen sind.

7. Pendeleinrichtung nach einem der Ansprüche 1 bis 6,
**dadurch gekennzeichnet, dass** die Leitungsverbindung zwischen den Ölräumen (31, 42) der Zylinder (29) und des Druckmittelwandlers (38) einen Schlauch (37) aufweist.

8. Pendeleinrichtung nach einem der Ansprüche 1 bis 7,
**dadurch gekennzeichnet, dass** die Drosselstelle (36) der Anschluss an dem Zylinder (29) für den Schlauch (37) ist.

9. Maschine mit wenigstens einer Pendeleinrichtung nach einem der Ansprüche 1 bis 8.

10. Maschine nach Anspruch 9,
**dadurch gekennzeichnet, dass** die Maschine eine Kehlmaschine ist.
